# EUROPEAN PATENT SPECIFICATION

(11) **EP 1 303 214 B1**
(45) Date of publication and mention of the grant of the patent: **05.10.2005**
(21) Application number: 01957765.9
(22) Date of filing: 25.07.2001
(51) Int. Cl.: A61B 5/04

(54) **AN ELECTROMEDICAL ELECTRODE WITH A SNAP CONNECTING MEANS**
ELEKTROMEDIZINISCHE ELEKTRODE MIT EINEM SCHNAPPVERBINDUNGSELEMENT
ELECTRODE ELECTROMEDICALE DOTEE D'UN MOYEN DE CONNEXION IMMEDIATE

(30) Priority: 25.07.2000 DK 200001132
(43) Date of publication of application: 23.04.2003
(73) Proprietor: MEDICOTEST A/S, 3650 Olstykke (DK)
(72) Inventor: BASSOE, Thomas, DK-4632 Bjaeverskov (DK); MADSEN, Torben, DK-3450 Allerod (DK); NIELSEN, Brian, DK-4700 Naestved (DK)
(74) Representative: Olsen, Lau Lund
(86) International application number: PCT/DK2001/000516
(87) International publication number: WO 2002/007597

(56) References cited:
- GB-A- 1 533 449
- US-A- 3 841 312
- US-A- 4 957 453

## Description

### Background of the invention

The present invention relates to an electromedical electrode comprising a plate-shaped part having a portion for establishing contact with the skin of a patient, a flange part connected with the plate-shaped part, snap connecting means provided on said flange part and including a stud portion for establishing connection to a lead wire connector means of a measuring apparatus, a base portion formed integrally with the stud portion, and fastening means for fastening the snap connecting means to the electrode, said snap connecting means being positioned at a distance from the skin contact establishing portion, and a conductive area extending between said skin contact establishing portion and the snap connecting means.

Electrodes establishing electrical contact between the skin of a patient and measuring apparatus are well-known in the art, and are e.g. used for measuring electrical signals generated in or by the body. For instance, the functions of the heart may be registered by recording the signals of the heart on an electrocardiogram (ECG).

The connection between the electrode and the measuring apparatus is provided by means of a lead wire having at one end a connector means which is coupled to the electrode in a suitable manner, most often by a snap connecting means fastened to the electrode.

US-A-3 841 312 discloses an electrode provided with a door knob shaped element serving to provide electrical contact to an elctrode connector. The door knob shaped element is formed integrally with an elongated member by means of a stud, and the elongated member is formed integrally with a circular flange. In the mounted position on the electrode, the elongated member extends through an electrically conductive gel pad and an adhesive pad, so that the circular flange is in contact with the electrically conductive gel pad. A separately formed apertured metal ring is positioned around the elongated member and has slightly bent metal tabs in contact with the elongated member, thereby holding securely in place the adhesive pad and the electrically conductive gel pad between the flange of the apertured metal ring and the flange of the stud.

Another example of an electrode having a snap connecting means which is offset from the skin contact establishing portion of the electrode is disclosed in EP patent No. 0 210 184. The snap connecting means of the electrode of this document is a two-piece construction in which a pin part comprising the stud and base portions is connected with a fastening means in the form of a rivet. During assembly of the electrode, the pin part and the rivet are placed on a respective side of the flange part of the electrode and are subsequently driven into interlocking engagement with each other. As both of these parts, i.e. the pin part and the fastening means are formed of an electrically conductive material, the electrical contact between the conductive area and the stud portion is established by this engagement. Due to the material consumption and the assembly operation required, the production costs are, however, undesirably high.

### Summary of the invention

With this background it is an object of the present invention to provide an electrode which is simple and inexpensive to produce, and which at the same time provides for a secure fastening of the snap connecting means and good electrical contact between the conductive area and the stud portion.

This object is met by an electrode according to claim 1.

By forming the fastening means integrally with the remaining portions of the snap connecting means, the material required is substantially reduced and the production costs are thus reduced. A safe fastening of the snap connecting means is provided by the folded legs, and the good electrical contact is provided by the abutment of the legs against the conductive area.

In a preferred embodiment, the legs are folded to a position in which the flange part and the conductive area are sandwiched between the base portion and the legs. In this way, it is by folding the legs possible to squeeze the flange part and the conductive area situated between the base portion and the legs, whereby a higher contact pressure and consequently a better electrical conductivity between the legs and the conductive area are obtained.

In an advantageous embodiment of the invention, the legs are folded away from each other to the position in which they abut the conductive area. In this manner, the contact points between the snap connecting means and the flange part with the conductive area are spread over a larger area. Further, it is possible to obtain a larger contact area by folding the legs outwards instead of inwards. Consequently, a more stable attachment of the snap connecting means to the electrode can be achieved. As a result, the snap connecting means is more resistant against impacts during handling of the electrode, for instance when the wire connected to the snap connecting means is pulled in different directions. Thereby a good electrical contact between the snap connecting means and the conductive area is ensured.

In an advantageous embodiment very simple to manufacture, the snap connecting means is punched out of a thin sheet material.

In an embodiment of the invention, flaps punched out of the sheet material and pointing away from each other are bent at a first position in a direction towards each other to form a double layer of the sheet material, said double layer forming part of the base portion, and the flaps are bent at a second position in a direction pointing away from the base portion to form the legs. In this way, it is in the form of the double layer of sheet material possible by simple punching and bending operations to obtain an outwardly protruding part of the base portion corresponding to each leg, so that the leg may be folded outwards to jam the flange part and the conductive area of the electrode between said part of the base portion and the leg.

In another embodiment, flaps punched out of the sheet material between remaining parts of the sheet material and pointing away from each other are bent in a direction pointing away from the base portion to form the legs, and said remaining parts of the sheet material form part of the base portion. This embodiment is very simple to manufacture, as only one punching operation and one bending operation are necessary. If the flange part is rather stiff, it may be jammed between the legs and said remaining parts of the sheet material.

If however, the flange part is flexible, advantageously parts of the sheet material forming the base portion are bent to form a double layer of the sheet material and to cover at least partially the holes in the base portion resulting from the punched out flaps. The sheet material covering said holes form an abutment, against which the flange part may be squeezed by the legs and thereby be secured firmly.

In yet another embodiment, the legs are folded-in to a position in which they abut said conductive area, whereby very little material is used.

Two legs may be provided which are positioned substantially diametrically opposite each other.

Advantageously, three or four legs are provided which in their folded positions are pointing in directions intersecting substantially at equal angles. This enhances the stability of the fastening means.

Each of said legs may be inserted through a separate corresponding punched hole in the flange part of the electrode, whereby a minimum of material is cut out of the flange part, ensuring stability of this.

In an advantageous embodiment very simple to manufacture, all of said legs are inserted through one single punched hole in the flange part of the electrode. The insertion of all the legs in only one hole may be easier, especially if the legs are inclined towards each other.

In a further advantageous embodiment, the single punched hole in the flange part has the shape of a polygon and has a straight side corresponding to each leg, respectively. This further enhances the stability of the fastening, as the legs may abut the edges of the hole.

Advantageously, edges of the legs and/or edges of the base portion are provided with teeth for better grip. The teeth may be directed towards the _flange part in the mounted position of the snap connecting means on the flange part.

The invention also relates to a method of manufacturing a snap connector for fastening on a flange part of an electromedical electrode for establishing electrical contact between a wire and a conductive area on the electrode. According to the invention, the method is characterized by punching out the snap connector of one single piece of sheet metal, whereby a substantially ball-shaped upper stud portion is formed for connection with the wire, and by punching out flaps to be folded as legs for fastening the connector to the flange part of the electrode. In this way, the above advantages are obtained.

The invention further relates to a method of fastening a snap connector on a flange part of an electromedical electrode for establishing electrical contact between a wire and a conductive area on the electrode. According to the invention, the method is characterized by introducing legs of the snap connector in one or more holes in the flange part of the electrode, and by bending the legs to abut the conductive area on the electrode.

### Brief description of the drawings

Examples of the invention will now be explained below with reference to the very schematic drawing, in which
Fig. 1 is an exploded view of an embodiment of the electrode according to the invention;
Fig. 2 is a sectional view of the electrode shown in Fig. 1 along the line II-II in Fig. 1;
Fig. 3 is a top view of a second embodiment of the snap connecting means according to the invention, before bending of flaps to form the legs;
Fig. 4 is a sectional view of the snap connecting means shown in Fig. 3, but after bending the flaps to form the legs;
Fig. 5 is a sectional view corresponding to Fig. 4, but after attachment of the snap connecting means to an electrode;
Fig. 6 is a top view of a third embodiment of the snap connecting means according to the invention, before bending of flaps to form the legs; and
Fig. 7 is a sectional view along the line VII-VII in Fig. 6, but after attachment of the snap connecting means to an electrode.

### Detailed description of the invention

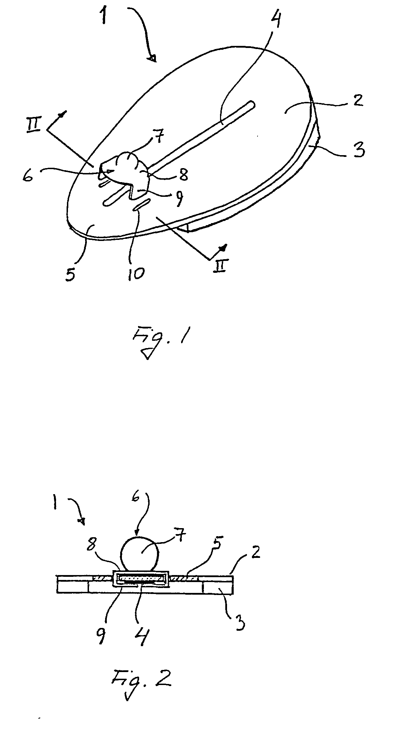

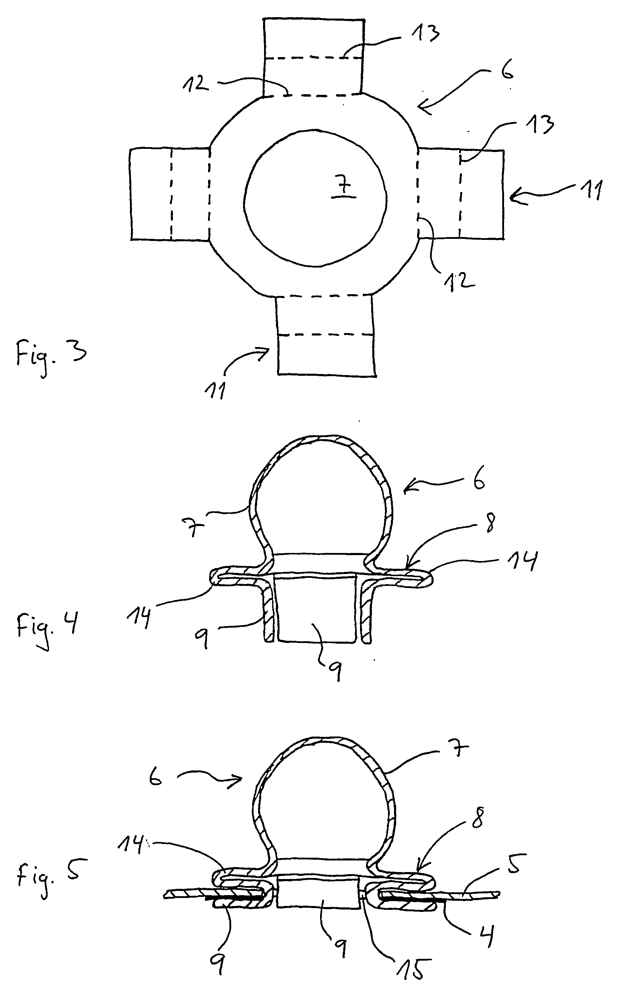

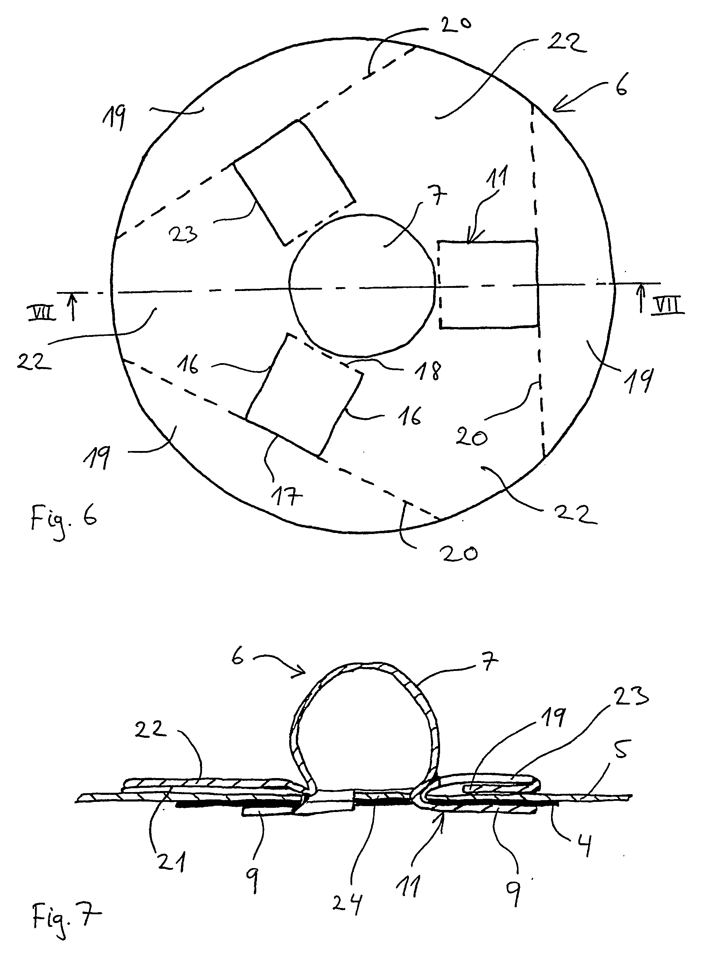

Referring to Figs. 1 and 2, an electromedical electrode generally designated 1 comprises a plate-shaped part 2 having on the side which in a position of use is intended to adhere to the skin of a patient a plate 3 of e.g. foam material. At a central location of the plate-shaped part, a hole (not shown) is provided for receiving an electrically conductive material, such as a paste-like electrolyte, thus providing a portion for establishing electrical contact with the skin. In a supply condition, the underside of the foam plate 3 may be covered by a protective layer (not shown) of e.g. a film material and which is removed prior to use.

The skin contact establishing portion of the plate-shaped part 2 is in electrical connection with a conductive area 4 which is shown on the upper side of the electrode in Fig. 1 for reasons of clarity only and which in the embodiment shown is designed as a substantially strip-shaped leading strip but which may have any appropriate shape. The conductive area 4 extends between the skin contact establishing portion to a flange part 5 of the electrode. The flange part 5 may be formed integrally with the plate-shaped part 2 as shown in the Figures, or be provided as a separate part which is connected with the plate-shaped part in any suitable manner. For instance, the upper side of the electrode, i.e. the flange part 5 and the part covering the foam plate 3 of the plate-shaped part 2 may be formed as a print which is punched out from a film material blank. The conductive area 4 may be formed by carbon which is printed on the blank or comprise a silver strip or any other suitable material.

On the flange part 5, a snap connecting means generally designated 6 is provided. The snap connecting means comprises a stud portion 7 which is formed integrally with a base portion 8 and which is intended for snap engagement with connector means of a wire of a measuring apparatus. The lead wire connector means may e.g. comprise a fitting or coupling member as disclosed in Applicants' above-mentioned EP patent No. 0 210 184 for receiving a banana plug, or be designed as a female connector means having a hole for snap engagement with the stud portion 7 of the snap connecting means 6.

Fastening means for securing the snap engagement means to the flange part 5 is provided by a plurality of legs, in the embodiment shown in Fig. 1 two legs 9 which are positioned substantially diametrically opposite each other. During assembly of the electrode 1, the legs 9 are inserted through a corresponding hole 10 in the flange part 5, and are subsequently folded-in to abut against the conductive area 4 as shown in Fig. 2.

The flange part 5 may on its underside be provided with a layer (not shown) of e.g. film material which covers i.a. the conductive area 4 and the folded-in legs 9 of the snap connecting means in order to prevent or reduce interference from the skin of the patient.

Fig. 3 to 5 show another embodiment of the snap connecting means 6 at different stages of the manufacturing process. Fig. 3 is a top view of the means 6 punched out of a peace of thin sheet metal. At this stage, the means has four flat flaps 11 pointing in different directions at right angles and an upstanding hollow, ball-like stud portion 7. Fig. 4 shows an axial section through the snap connecting means of Fig. 3 after first bending the flaps 11 in a direction towards the central axis of the means 180 degrees along the dotted lines 12 of Fig. 3 and then bending the flaps 90 degrees along the dotted lines 13 of Fig. 3 in the opposite direction in order to form outwards protruding parts 14 of the base portion 8 as well as the downward legs 9. Fig. 5 shows the snap connecting means 6 after fastening on the flange part 5 by insertion of the legs 9 through one square hole 15 in the flange part 5 and subsequent bending of the legs 9 to the position shown where they abut the conductive area 4 on the lower face of the flange part 5.

Fig. 6 is a top view of a third embodiment of the snap connecting means 6 after punching it out of a peace of thin sheet metal. At this stage, the means has three flat flaps 11 pointing in different directions forming an angle of 120 degrees with each other and an upstanding hollow, ball-like stud portion 7. The flaps 11 are cut free from the surrounding sheet material along their two opposite side edges 16 and their outward end edge 17, but connected with the surrounding material at their inward end edge which is indicated by the dotted line 18. Fig. 7 shows the snap connecting means 6 after fastening on the flange part 5. First, the flaps 11 have been bent 90 degrees or more downwards along the lines 18 of Fig. 6 and subsequently outer parts 19 of the surrounding sheet material have been bent downwards 180 degrees along the dotted lines 20 of Fig. 6 to abut a lower side 21 of parts 22 of sheet material situated between holes 23 resulting from the bent down flaps 11. As a result, part of the holes 23 is covered by these outer parts 19 of sheet material so that an abutment is formed against which the material of the flange 5 is pressed by the legs 9 after insertion of these through a triangular hole 24 in the flange 5 and bending of the legs 9 outwards to abut the conductive area 4 on the lower side of the flange 5. The triangular shape of the hole 24 corresponds to the three dotted lines 18 along which the legs 9 are bent and thereby assures good securing of the snap connecting means 6 in the direction of the plane of the flange 5. If the material of the flange 5 is relatively stiff, the folded-in parts 19 may be omitted.

It should be mentioned, that corresponding elements are referred to by corresponding reference numerals in the different embodiments shown. For reasons of clarity, the sheet material of the snap connecting means as well as the material of the flange 5 are shown very thick in the figures.

The snap connecting means 6 is manufactured by means of techniques know per se, and of any suitable material, such as nickel-plated or silver-plated brass or a steel material which is preferably corrosion-resistant and acid-proof.

The invention should not be regarded as being limited to the embodiments described in the above but various modifications of the electrode may be carried out without departing from the scope of the appended claims. For instance, all shown embodiments may be produced with any suitable number of legs 9, and the legs may be inserted through separate holes or one common hole in the flange 5.

## Claims

1. An electromedical electrode (1) comprising
a plate-shaped part (2) having a portion for establishing contact with the skin of a patient,
a flange part (5) connected with the plate-shaped part (2),
snap connecting means (6) provided on said flange part (5) and including a stud portion (7) for establishing connection to a lead wire connector means of a measuring apparatus, a base portion (8) formed integrally with the stud portion (7), and fastening means for fastening the snap connecting means to the plate-shaped part (2), said snap connecting means being positioned at a distance from the skin contact establishing portion, and
a conductive area (4) extending between said skin contact establishing portion and the snap connecting means (6),
the fastening means of said snap connecting means (6) being formed integrally with the base portion and comprising a plurality of folded legs (9) which protrude from said base portion, and said legs being folded to a position in which they abut against said conductive area (4).

2. An electrode according to claim 1, **characterized in that** the legs are folded to a position in which the flange part (5) and the conductive area (4) are sandwiched between the base portion (8) and the legs (9).

3. An electrode according to claim 1 or 2, **characterized in that** the legs (9) are folded away from each other to the position in which they abut the conductive area (4).

4. An electrode according to any of the preceding claims, **characterized in that** the snap connecting means (6) is punched out of a thin sheet material.

5. An electrode according to claim 4, **characterized in that** flaps (11) punched out of the sheet material and pointing away from each other are bent at a first position (12) in a direction towards each other to form a double layer of the sheet material, said double layer forming part of the base portion (8), and **in that** the flaps (11) are bent at a second position (13) in a direction pointing away from the base portion (8) to form the legs (9).

6. An electrode according to claim 4, **characterized in that** flaps (11) punched out of the sheet material between remaining parts (22) of the sheet material and pointing away from each other are bent in a direction pointing away from the base portion (8) to form the legs (9), and **in that** said remaining parts (22) of the sheet material form part of the base portion (8).

7. An electrode according to claim 6, **characterized in that** parts (19) of the sheet material forming the base portion (8) are bent to form a double layer of the sheet material and to cover at least partially the holes (23) in the base portion (8) resulting from the punched out flaps (11).

8. An electrode according claim 1 or 2, **characterized in that** the legs (9) are folded against each other to the position in which they abut said conductive area (4).

9. An electrode according to any of the preceding claims, **characterized in that** two legs (9) are provided which are positioned substantially diametrically opposite each other.

10. An electrode according to any one of the claims 1 to 8, **characterized in that** three or four legs (9) are provided which in their folded positions are pointing in directions intersecting substantially at equal angles.

11. An electrode according to any one of the preceding claims, **characterized in that** each of said legs (9) is inserted through a separate corresponding punched hole (10) in the flange part (5) of the electrode.

12. An electrode according to any one of the claims 1 to 10, **characterized in that** all of said legs (9) are inserted through one single punched hole (15, 24) in the flange part (5) of the electrode.

13. An electrode according to claim 12, **characterized in that** the single punched (15, 24) hole in the flange part (5) has the shape of a polygon and has a straight side corresponding to each leg (9), respectively.

14. An electrode according to any one of the preceding claims, **characterized in that** edges of the legs and/or edges of the base portion are provided with teeth.

15. An electrode according to claim 14, **characterized in that** the teeth are directed towards the flange part (5) in the mounted position of the snap connecting means (6) on the flange part.

16. An electrode according to any one of the preceding claims, **characterized in that** said snap connecting means (6) comprises nickel-plated or silver-plated brass.

17. An electrode according to any one of the claims 1 to 13, **characterized in that** said snap connecting means (6) comprises corrosion-resistant and/or acid-proof steel.

18. A method of manufacturing a snap connector (6) for fastening on a flange part (5) of an electromedical electrode (1) for establishing electrical contact between a wire and a conductive area (4) on the electrode, by punching out the snap connector of one single piece of sheet metal, whereby a substantially ball-shaped upper stud portion (7) is formed for connection with the wire, and by punching out flaps (11) to be folded as legs (9) for fastening the connector (6) to the flange part (5) of the electrode.

19. A method of fastening a snap connector (6) on a flange part (5) of an electromedical electrode (1) for establishing electrical contact between a wire and a conductive area (4) on the electrode, by introducing legs (9) of the snap connector in one or more holes (10, 15, 24) in the flange part (5) of the electrode, and by bending the legs to abut the conductive area on the electrode.

## Patentansprüche

1. Elektromedizinische Elektrode (1) umfassend einen plattenförmigen Teil (2) mit einem Abschnitt zum Erzeugen von Kontakt mit der Haut eines Patienten,
einen mit dem plattenförmigen Teil (2) verbundenen Flanschteil (5),
auf dem Flanschteil (5) vorgesehene Schnappverbindungsmittel (6) und umfassend einen Gerippeabschnitt (7) zum Anschluss an ein Zuleitungsdrahtverbindungsmittel eines Messgeräts, einen mit dem Gerippeabschnitt (7) integral ausgebildeten Basisabschnitt (8), und Mitteln zum Befestigen des Schnappverbindungsmittels an den plattenförmigen Teil (2), welches Schnappverbindungsmittel in einem Abstand von dem Hautkontakt erzeugenden Teil angeordnet ist, und
einen sich zwischen dem Hautkontakt erzeugenden Teil und dem Schnappverbindungsmittel (6) erstrecktenden leitfähigen Bereich (4),
wobei die Befestigungsmittel des Schnappverbindungsmittels (6) mit dem Basisabschnitt integral ausgebildet sind und eine Mehrzahl aus dem Basisabschnitt hervorstehender gefalteter Schenkel (9) umfassen, und erwähnte Schenkel in eine Stellung gefaltet sind, in der sie gegen erwähnten leitfähigen Bereich (4) anliegen.

2. Elektrode nach Anspruch 1, **dadurch gekennzeichnet, dass** die Schenkel in eine Stellung gefaltet sind, in welcher der Flanschteil (5) und der leitfähige Bereich (4) zwischen dem Basisabschnitt (8) und den Schenkeln (9) eingeklemmt sind.

3. Elektrode nach Anspruch 1 oder 2, **dadurch gekennzeichnet, dass** die Schenkel (9) voneinander weg gefaltet sind in die Stellung, in der sie an dem leitfähigen Bereich (4) anliegen.

4. Elektrode nach einem der vorhergehenden Ansprüche, **dadurch gekennzeichnet, dass** das Schnappverbindungsmittel (6) aus einem dünnen Blechmaterial ausgestanzt ist.

5. Elektrode nach Anspruch 4, **dadurch gekennzeichnet, dass** aus dem Blechmaterial ausgestanzte und voneinander weg weisende Klappen (11) in einer ersten Stellung (12) in eine Richtung zueinander zu einer einen Teil des Basisabschnitts (8) bildenden Doppelschicht des Blechmaterials gebogen sind, und dass die Klappen (11) in einer zweiten Stellung (13) in eine von dem Basisabschnitt (8) weg weisende Richtung zur Bildung der Schenkel (9) gebogen sind.

6. Elektrode nach Anspruch 4, **dadurch gekennzeichnet, dass** die aus dem Blechmaterial zwischen übrigen Teilen (22) von Blechmaterial ausgestanzten und voneinander weg weisenden Klappen (11) zur Bildung der Schenkel (9) in eine von dem Basisabschnitt (8) weg weisende Richtung gebogen sind, und dass die übrigen Teile (22) des Blechmaterials einen Teil des Basisabschnitts (8) bilden.

7. Elektrode nach Anspruch 6, **dadurch gekennzeichnet, dass** die den Basisabschnitt (8) bildenden Teile (19) des Blechmaterials derart gebogen sind, dass sie eine Doppelschicht des Blechmaterials bilden und zumindest teilweise die aufgrund der ausgestanzten Klappen (11) entstandenen Löcher (23) im Basisabschnitt (8) decken.

8. Elektrode nach Anspruch 1 oder 2, **dadurch gekennzeichnet, dass** die Schenkel (9) zueinander in die Stellung gefaltet sind, in der sie an dem leitfähigen Bereich (4) anliegen.

9. Elektrode nach einem der vorhergehenden Ansprüche, **dadurch gekennzeichnet, dass** zwei im wesentlichen diametral entgegengesetzt angeordnete Schenkel (9) vorgesehen sind.

10. Elektrode nach einem der Ansprüche 1-8, **dadurch gekennzeichnet, dass** drei oder vier Schenkel (9) in ihren gefalteten Stellungen in Richtungen zeigen, die sich bei im wesentlichen gleichen Winkeln schneiden.

11. Elektrode nach einem der vorhergehenden Ansprüche, **dadurch gekennzeichnet, dass** jeder Schenkel (9) durch ein separates, entsprechend ausgestanztes Loch (10) in den Flanschteil (5) der Elektrode eingesetzt ist.

12. Elektrode nach einem der Ansprüche 1-10, **dadurch gekennzeichnet, dass** alle Schenkel (9) durch ein einziges ausgestanztes Loch (15, 24) in den Flanschteil (5) der Elektrode eingesetzt sind.

13. Elektrode nach Anspruch 12, **dadurch gekennzeichnet, dass** das einzige im Flanschteil (5) ausgestanzte Loch (15, 24) die Form eines Polygons hat und eine jedem Schenkel (9) entsprechende gerade Seite aufweist.

14. Elektrode nach einem der vorhergehenden Ansprüche, **dadurch gekennzeichnet, dass** Kanten der Schenkel und/oder Kanten des Basisabschnitts mit Zähnen versehen sind.

15. Elektrode nach Anspruch 14, **dadurch gekennzeichnet, dass** die Zähne in der montierten Stellung des Schnappverbindungsmittels (6) auf dem Flanschteil gegen den Flanschteil (5) gerichtet sind.

16. Elektrode nach einem der vorhergehenden Ansprüche, **dadurch gekennzeichnet, dass** das Schnappverbindungsmittel (6) vernickeltes oder versilbertes Messing umfasst.

17. Elektrode nach einem der Ansprüche 1-13, **dadurch gekennzeichnet, dass** das Schnappverbindungsmittel (6) korrosionsbeständigen und/oder säurebeständigen Stahl umfasst.

18. Verfahren zur Herstellung einer Schnappverbindung (6) zum Befestigen auf einem Flanschteil (5) einer elektromedizinischen Elektrode (1) zum Erzeugen von elektrischem Kontakt zwischen einem Draht und einem leitfähigen Bereich (4) auf der Elektrode durch Ausstanzen der Schnappverbindung aus einem einzigen Stück Blechmaterial, wobei ein im wesentlichen kugelförmiger oberer Gerippeabschnitt (7) zum Verbinden mit dem Draht ausgebildet ist, und durch Austanzen von Klappen (11), die zwecks Befestigen des Konnektors (6) auf dem Flanschteil (5) der Elektrode wie Schenkel (9) gefaltet werden.

19. Verfahren zum Befestigen eines Schnappkonnektors (6) auf einem Flanschteil (5) einer elektromedizinischen Elektrode (1) zum Erzeugen von elektrischem Kontakt zwischen einem Draht und einem leitfähigen Bereich (4) auf der Elektrode durch Einsetzen von Schenkeln (9) des Schnappkonnektors in ein Loch oder mehrere Löcher (10, 15, 24) im Flanschteil (5) der Elektrode, und durch Biegen der Schenkel zum Anliegen an dem leitfähigen Bereich der Elektrode.

## Revendications

1. Electrode électromédicale (1) comprenant
une portion formée en plaque (2) présentant une portion pour établir un contact avec la peau d'un patient,
une portion de bride (5) reliée à la portion formée en plaque (2),
des moyens de connexion immédiate (6) pourvus sur ladite portion de bride (5) et comprenant une portion de goujon (7) pour établir une connexion avec un moyen connectif à fil conducteur d'un appareil de mesure, une portion de base (8) formée d'une seule pièce avec la portion de goujon (7), des moyens de fixation pour fixer les moyens de connexion immédiate à la portion formée en plaque (2), moyens de connexion immédiate qui sont placés à une distance de la portion établissant contact avec la peau, et
une zone conductrice (4) s'étendant entre ladite portion établissant un contact avec la peau et les moyens de connexion immédiate (6),
le moyen de fixation desdits moyens de connexion immédiate (6) étant formé d'une seule pièce avec la portion de base et comprenant une multitude de pieds pliés (9) faisant saillie de ladite portion de base, et lesdits pieds étant pliés à une position dans laquelle ils s'appuient sur ladite zone conductrice (4).

2. Electrode selon la revendication 1, **caractérisée en ce que** les pieds sont pliés à une position dans laquelle la portion de bride (5) et la zone conductrice (4) sont sandwichées entre la portion de base (8) et les pieds (9).

3. Electrode selon la revendication 1 ou 2, **caractérisée en ce que** les pieds (9) sont pliés l'un loin de l'autre à une position dans laquelle ils s'appuient sur la zone conductrice (4).

4. Electrode selon l'une quelconque des revendications précédentes, **caractérisée en ce que** le moyen de connexion immédiate (6) est découpé d'un matériau de lame mince.

5. Electrode selon la revendication 4, **caractérisée en ce que** des volets (11) découpés du matériau de lame et orientés l'un loin de l'autre sont pliés à une première position (12) dans une direction l'un vers l'autre pour créer une double couche du matériau de lame, double couche qui fait partie de la portion de base (8), et **en ce que** les volets (11) sont pliés à une deuxième position (13) dans une direction détournée de la portion de base (8) pour créer les pieds (9).

6. Electrode selon la revendication 4, **caractérisée en ce que** des volets (11) découpés du matériau de lame entre des portions restantes (22) du matériau de lame et orientés l'un loin de l'autre sont pliés dans une direction détournée de la portion de base (8) pour créer les pieds (9), et **en ce que** lesdites portions restantes (22) du matériau de lame font partie de la portion de base (8).

7. Electrode selon la revendication 6, **caractérisée en ce que** les portions (19) du matériau de lame créant la portion de base (8) sont pliées à former une double couche du matériau de lame et à couvrir au moins partiellement les trous (23) dans la portion de base (8) résultant des volets découpés (11).

8. Electrode selon la revendication 1 ou 2, **caractérisée en ce que** les pieds (9) sont pliés l'un contre l'autre à la position dans laquelle ils s'appuient sur ladite zone conductrice (4).

9. Electrode selon l'une quelconque des revendications précédentes, **caractérisée en ce que** les deux pieds (9) sont pourvus dans une position où ils sont diamétralement opposés l'un à l'autre.

10. Electrode selon l'une des revendications 1 à 8, **caractérisée en ce que** trois ou quatre pieds (9) sont pourvus dans leurs positions pliées où ils sont orientés dans des directions s'entrecroisant essentiellement à des angles égaux.

11. Electrode selon l'une quelconque des revendications précédentes, **caractérisée en ce que** chacun desdits pieds (9) est inséré à travers un trou correspondant, séparé et découpé (10) dans la portion de bride (5) de l'électrode.

12. Electrode selon l'une quelconque des revendications 1 à 10, **caractérisée en ce que** tous lesdits pieds (9) sont insérés à travers un seul trou découpé (15, 24) dans la portion de bride (5) de l'électrode.

13. Electrode selon la revendication 12, **caractérisée en ce que** le seul trou découpé (15, 24) dans la portion de bride (5) présente respectivement la forme d'un polygone et un côté droit correspondant à chaque pied (9).

14. Electrode selon l'une quelconque des revendications précédentes, **caractérisée en ce que** des bords des pieds et/ou des bords de la portion de base sont pourvus de dents.

15. Electrode selon la revendication 14, **caractérisée en ce que** les dents sont orientées vers la portion de bride (5) dans la position montée des moyens de connexion immédiate (6) sur la portion de bride.

16. Electrode selon l'une des revendications précédentes, **caractérisée en ce que** ledit moyen de connexion immédiate (6) comprend le cuivre nickelé ou argentifère.

17. Electrode selon l'une quelconque des revendications 1 à 13, **caractérisée en ce que** ledit moyen de connexion immédiate (6) comprend l'acier résistant à la corrosion et/ou l'acier à haute résistance à l'acide.

18. Procédé permettant de produire un dispositif de connexion immédiate (6) à fixer sur une portion de bride (5) d'une électrode électromédicale (1) pour établir un contact électrique entre un fil et une zone conductrice (4) sur l'électrode par découpage du dispositif de connexion immédiate d'une seule pièce de métal en plaque, une portion supérieure de goujon essentiellement en boule (7) étant créée pour connexion avec le fil, et par découpage de volets (11) à plier comme des pieds (9) pour fixer le dispositif de connexion (6) à la portion de bride (5) de l'électrode.

19. Procédé permettant de fixer un dispositif de connexion immédiate (6) sur une portion de bride (5) d'une électrode électromédicale (1) pour établir du contact électrique entre un fil et une zone conductrice (4) sur l'électrode par l'introduction des pieds (9) du dispositif de connexion immédiate dans un ou plusieurs trous (10, 15, 24) dans la portion de bride (5) de l'électrode et par pliage des pieds à s'appuyer sur la zone conductrice sur l'électrode.
